# EUROPEAN PATENT APPLICATION

(11) **EP 3 398 609 A1**
(43) Date of publication of application: **07.11.2018**
(21) Application number: 16880201.5
(22) Date of filing: 24.10.2016
(51) Int. Cl.: A61K 38/24, C12N 15/16, C07K 14/59, C12N 15/62, C12N 15/53, C12N 15/87

(54) **PROCESS OF PRODUCING A RECOMBINANT EQUINE CHORIONIC GONADOTROPIN (RECG), VETERINARY COMPOSITION AND USE THEREOF**

(30) Priority: 28.12.2015 BR 102015326602
(71) Applicant: Ouro Fino Saúde Animal Participações S.A., CEP : 14140-000 Cravinhos SP (BR); Universidade de São Paulo - USP, 05508-220 São Paulo (BR)
(72) Inventor: SOGAYAR, Mari Cleide, 05586-090 São Paulo (BR); OLIVEIRA CARREIRA NISHIYAMA, Ana Claudia, 05539-080 São Paulo (BR); ALMEIDA DEMASI, Marcos Angelo, 04581-050 São Paulo (BR); CAMARGO, Lauren, 05586-001 São Paulo (BR); MALDONADO COELHO, Tatiane, 04313-002 São Paulo (BR)
(74) Representative: Pereira Garcia, João Luís
(86) International application number: PCT/BR2016/050267
(87) International publication number: WO 2017/112987

(57) **Abstract**

The present invention relates to the production of recombinant equine chorionic gonadotrophin (reCG), to a veterinary composition comprising reCG, and to the use of reCG or the composition comprising reCG. The reCG of the present invention has a glycosylation profile similar to that of commercially available chorionic gonadotrophins and exhibits the level of *in vivo* activity corresponding to eCG and similar to that of the FSH and LH hormones. The present invention has application in the field of biotechnology, more specifically, in the field of assisted-veterinary reproduction, being especially suitable for use in the treatment of veterinary conditions related to mammalian reproduction and ovulation, for the manufacture of diagnostic kits or as supplement for cell culture.

## Description

### Technical Field

The present invention relates to the production process of the recombinant equine chorionic gonadotropin, referred to herein as reCG or reCGβα, the reCG produced by the process of the present invention, a veterinary composition comprising reCG, and the use of reCG or composition comprising reCG.

The produced reCG presents glycosylation profile similar to that of commercially available chorionic gonadotropin and exhibits *in vivo* level of activity similar to that of luteinizing hormone (LH, English acronym for *Luteinizing Hormone*) and follicle stimulating hormone (FSH, English acronym for *Follicle Stimulating Hormone*).

The present invention has application in biotechnology, more particularly in the field of veterinary assisted reproduction, being especially suitable for use in the treatment of veterinary conditions related to reproduction and ovulation in mammals, for the manufacture of diagnostic kits or as a supplement for cell culture.

### History of the Invention

Currently, Brazil is in a privileged position as the largest producer and exporter of bovine meat in the world, making the livestock one of the national economic activities more important and profitable. This data emphasizes the importance of basic and technological research in cattle breeding, especially in the area of ovulation stimulating hormones, such as equine chorionic gonadotrophin (eCG).

Several hormones are used in assisted veterinary reproduction techniques, and in recent years, equine chorionic gonadotropin emerges as the first choice for the induction of ovulation in cows gestational anestrus, in fixed-time artificial insemination (FTAI) protocols, aiming to improve the fertility rate of cows created in extensive regime (SÁ FILHO et al., Equine chorionic gonadotropin and gonadotropin-releasing hormone enhance fertility in norgestomet-based, timed artificial insemination protocol in sucked Nellore (Bos indicus) cows. Theriogenology 2010; 73(5):651-8; FERREIRA et al., Effect of different doses of equine chorionic gonadotropin on follicular and lutteal dynamics and P/Al of high-producing Holstein cows. Anim Reprod Sci 2013; 140(1-2) 26-33; CARVALHO et al., Equine chorionic gonadotropin improves the efficacy of a timed artificial insemination protocol in buffalo during the nonbreeding season. Theriogenology 2013;79(3):423-8; BARREIROS et al., Dynamics of follicular growth and progesterone concentrations in cyclic and anestrous suckling Nelore cows (Bos indicus) treated with progesterone, equine chorionic gonadotropin, or temporary calf removal. Theriogenology 2014; 81(5):651-6).

The ECG is used specially in beef cows reared in extensive handling and has been used also in superovulation treatment of cows and heifers as an effective treatment option for fractious animal (MAPLETOFT et al., Recent advances in the superovulation in cattle. Reprod Nutr Dev 2002; 42(6):601-11), whereas in conventional hormonal treatments in cattle raising adopt the use of eight doses of commercial preparations from purified porcine FSH -Folltropin-V©, Bioniche© (data obtained in the product leaflet).

The ECG is secreted by the equine uterus equine, more specifically by the endometrial cups, which are formed around the fortieth day of pregnancy and remained up around the eighty-fifth day. The secretion of this hormone, which has similar biological action as both FSH and LH, stimulates the development of ovarian follicles in pregnant mare. Some of these follicles ovulate, but most becomes luteinized follicles due to action similar to LH. Such additional *corpus luteum* produce progesterone required to maintain the mare pregnant pregnancy in the mare (HAFEZ E HAFEZ, Reprodução Animal. Editora Manole 2004; volume único).

The ECG currently used for animal breeding is purified from plasma of pregnant mares (CHRISTAKOS E BAHL, Pregnant mare serum gonadotropin. Purification and physicochemical, biological, and immunological characterization. J Biol Chem 1979; 254(10):4253-61; MAPLETOFT et al., Recent advances in the superovulation in cattle. Reprod Nutr Dev 2002; 42(6):601-11) and marketed as Folligon (Intervet); SincroeCG (Ourofino) e Novormon (Coopers), and there is no commercially available recombinant ECG.

This unique gonadotropin has the capability, in heterologous species, to bind to receptors of both FSH and LH, and to cause the stimulation of folliculogenesis (STEWART et al., Pregnant mare serum gonadotrophin: ratio of follicle-stimulating hormone and luteinizing hormone activities measured by radioreceptor assay. J Endocrinol 1976; 71(3):471-82; COMBARNOUS, Original hypothesis on the specificity of the binding of glycoprotein hormones to their receptors. C R Seances Acad Sci III 1981). Its prolonged plasma half-life, due to their high amount of sugars, (MCINTOSH et al., Pregnant mare serum gonadotrophin: rate of clearance from the circulation of sheep. J Reprod Fertil 1975; 44(1):95-100), allows that a single application of this hormone be effective to induce ovulation, saving thus the time spent handling, and also reducing possible stresses of animals undergoing superovulation (KIMURA et al., Successful superovulation of cattle by a single administration of FSH in aluminum hydroxide gel. Theriogenology 2007; 68(4):633-9).

Besides the extraction of gonadotropins be a laborious and costly task, the lack of biosafety of the resulting material is quite disturbing, since any attempt at purification it preserves the glycoprotein hormones demonstrates a limited capacity of resistant-virus destruction (THARASANIT et al., Effects of recombinant human follicle stimulating hormone of follicle development and ovulation in the mare. Theriogenology 2006; 65(6):1071-81). Moreover, because it is a biological extract, there is great variability in glycoprotein activity among lots of eCG, contributing in this way to the variability in response and quality of obtained embryos (WILSON et al., Superovulation of cattle with a recombinant-DNA bovine follicle stimulating hormone. Animal Reproduction Science 1993;33(1-4):71-82).

In light of the above and taking into account the fact that the source of the eCG is limited (blood of pregnant mares), and also that there are bioethical factors related to obtaining this material, a source of eCG is desired to be unlimited, ethics, and more practical and reproducible achievement, ensuring uninterrupted supply to a growing consumer market of this hormone.

The recombinant gonadotropins have maximum bio-security, better performance in the results, no interference of other hormones and few contaminants, and, thus, used widely in human Reproductive Medicine, representing great interest for the veterinary assisted reproduction. Recombinant technology has proved possible to produce complex proteins reproducibly. However, the structure of gonadotropins is difficult to reproduce because, besides the presence of several post-translational modifications, gonadotropins are composed of two transcripts chains and translated independently which are subsequently combined (LOUMAGEL et al., Human follicle-stimulating hormone produced by recombinant DNA technology: a review for clinicians. Human Reproduction 1995; v. 1 p. 188-199).

The process of the present invention can also be applied to other forms of eCG that are not fused such as, for example, separately expressed chains, or changes of the protein to increase the circulation time of the molecule, such as, for example, changes as PEGlation.

The α subunit of eCG, consisting of 96 amino acids, exhibits two complex N-linked oligosaccharide chains, located at the asparagines residues 56 and 82. However, the carbohydrate chains of the two hormones exhibit differences in their structures: the α subunit of eLH has two biantennary N-glycans terminated in N-sulfated acetylgalactosamines (SO4-4-GalNAc), whereas the N-glycans attached to the α subunit of eCG are terminated in sialic acid in the α 2,3- and α2,6-links (DAMM et al., Structure determination of the major N- and O-linked carbohydrate chains of the beta subunit from equine chorionic gonadotropin. Eur J Biochem. 1990 Apr 20;189(1):175-83.), possibly extended with lactosamine repeats (BOUSFIELD et al., Differential effects of alpha subunit Asparagine56 oligosaccharide structure on equine lutropin and follitropin hibrid conformation and receptor-binding activity. Biochemistry. 2004 Aug 24;43(33):10817-33). The β subunit of eCG, composed of 149 amino acid residues (SUGINO et al., Structural studies on equine glycoprotein hormones. Amino acid sequence of equine chorionic gonadotropin beta-subunit. J Biol Chem. 1987 Jun 25;262(18):8603-9), has a single chain of N-glycan located at Asn13, ending with sulfated GalNAc in eLH and α2,3Gal sialylated in eCG (SMITH et al., Equine lutropin and chorionic gonadotropin bear oligosaccharides terminating with SO4-4-GalNAc and Sia alpha 2,3Gal, respectively. J Biol Chem. 1993 Jan 15;268(2):795-802; MATSUI et al., Structural analysis of N-linked oligosaccharides of equine chorionic gonadotropin and lutropin beta-subunits. Biochemistry. 1994 Nov 29;33(47):14039-48). Both β subunits have up to 12 O-glycans extended with sialylated poly N-acetyllactosamine (α2,3) located in the serine or threonine of the carboxy-terminal peptide (CTP) of 28 amino acid residues (HOKKE et al., Structure determination of the disialylated poly-(N-acetyllactosamine)-containing O-linked carbohydrate chains of equine chorionic gonadotropin. Glycoconj J. 1994 Feb;11(1):35-41; BOUSFIELD et al., Identification of twelve O-glycosylation sites in equine chorionic gonadotropin beta and equine luteinizing hormone ss by solid-phase Edman degradation. Biol Reprod. 2001 Jan;64(1):136-47.). As eLH and eCG have identical polypeptide chains, the difference between the structure of N- and O-glycans explains the difference between the molecular weights and biological activity of these hormones: eCG presents an exceptional circulatory half-life compared to eLH (BOUSFIELD et al., Identification of twelve O-glycosylation sites in equine chorionic gonadotropin beta and equine luteinizing hormone ss by solid-phase Edman degradation. Biol Reprod. 2001 Jan;64(1):136-47). Removal of the terminal sialic acid residues from glycan chains of eCG dramatically decreases the *in vivo* activity of this glycoprotein since its half-life is reduced from six days to about 1-2 hours (MARTINUK et al., Effects of carbohydrates on the pharmacokinetics and biological activity of equine chorionic gonadotropin in vivo. Biol Reprod. 1991 Oct;45(4):598-604).

In the literature, many authors have tried to obtain recombinant equine chorionic gonadotropin, and this was cloned and expressed in insect cells (LEGARDINIER et al., Biological activities of recombinant equine luteinizing hormone/chorionic gonadotropin (eLH/CG) expressed in Sf9 and Mimic insect cell lines. Journal of Molecular Endocrinology 2005; 34:47-60); secreted in milk of transgenic rabbits (GALET et al., Expression of an in vitro biologically active equine LH/CG without C-terminal peptide (CTP) and/or beta26-110 disulphide bridge. J Endocrinol, 2001; 167(1):117-24); expressed in mouse cells (GALET et al., Expression of a single beta alpha chain protein of equine LH/CG in milk of transgenic rabbits and its biological activity. Mol Cell Endocrinol, 2001; 174(1-2):31-40); and modified and expressed as deglycosylated forms in CHO cells (Chinese hamster ovary cells). Only for the first two systems, *in vivo* biological activity assays were conducted, resulting in no activity, a fact resulting from the difference in glycan chains added to eCG by these expression systems.

Additionally, the prior art teaches recombinant gonadotropins production methods, but does not propose an effective way to obtain reCG in large quantities, and at the same time with the glycosylation profile similar to wild eCG ensuring *in vivo* reCG activity similar to that observed for wild eCG.

HESSER (HESSER, A survey of heterologous expression systems for the production of bovine follicle stimulating hormone and luteinizing hormone. All Dissertations 2011; Paper 692) describes the production of gonadotropins, more specifically, follicle-stimulating hormones in CHO cells.

FURUHASHI *et al.* (FURUHASHI et al., Fusing the carboxyterminal peptide of the chorionic gonadotropin (CG) beta subunit to the common alpha subunit: retention of O-linked glycosylation and enhanced in vivo bioactivity of chimeric human CG. Mol Endocrinol. 1995 Jan;9(1):5463) describes an hCG analog with a CTP-type polypeptide tail (English acronym for Carboxy-Terminal Peptide) and relates the importance of glycosylation for biological activity of the analog.

SUGAHARA *et al.* (SUGAHARA et al., Biosynthesis of a biological active single peptide chain containing the human common α and chorionic gonadotropin β subunits in tandem. Proc Natl Acad Sci USA 1995; 92:2041-2045) describes the biological activity of a single polypeptide chain with activity biological, containing a β subunit of hCG fused to an α subunit of gonadotropins.

Subsequently, SUGAHARA *et al.* (SUGAHARA et al., Expression of biological active fusion genes encoding the common alpha subunit and the follicle-stimulating hormone beta subunit: Role of a linker sequence. J Biol Chem 1996; 271:10445-10448) described the conversion of a FSH heterodimer for a single chain structure, and the impact of the absence of the linker sequence on secretion rate and said protein binding. Also, it shows that the alignment of α and β domains differ from the one presented in the form of heterodimer.

NARAYAN *et al.* (NARAYAN et al., Functional expression of yoked human chorionic gonadotropin in baculovirus-infected insect cells. Mol Endocrinol. 1995; 9(12):1720-6) describes the simultaneous expression of the α and β subunits of eCG in insect cells, whose expression is controlled by different promoters on the same recombinant baculovirus, yielding proteins which are *in vitro* biologically active.

SHIOTA *et al.* (SHIOTA et al., Production of recombinant eCG with potent FSH-like activity by site-directed mutagenesis. Nippon Yakurigaku Zasshi 1997; 110(1):59P-62P) describes recombinant gonadotropins with different structures in view of the coupled oligosaccharides and relates to the biological activity of the mutants or variants.

The document EP 0.974.599 describes a recombinant eCG variant and its corresponding DNA sequence, plus a pharmaceutical composition of said recombinant eCG for use as a veterinary drug. The disclosed variant form of the recombinant eCG has α and β chains of eCG fused in a single polypeptide chain having the *in vitro* activity of FSH- and LH-type. The inventors suggest their use as a medicine for animals but do not demonstrate: a) the similarity between the glycosylation profile of the obtained recombinant form and that of the wild-type form; and b) *in vivo* biological activity of the obtained recombinant form of eCG.

The document US 5.767.251 describes methods for generating recombinant human gonadotropins, among them hCG, hLH and hFSH, wherein the hormone composed of two different subunits are synthesized in the same cell by at least one expression vector, wherein the expression of each subunit is controlled by a different promoter. In this case, the biological activity of hCG is easily distinguished from the LH activity because, unlike what occurs in horses, the β chains of these two gonadotropins are encoded by distinct genes in humans.

The document US 5.047.335 describes a process for controlling glycosylation of recombinant proteins involving the use of genetically modified CHO line cells, such that to produce sialyltransferases, a class of glycosyltransferases. This additional production of sialyltransferases enables, for example, that recombinant glycoproteins produced on this line of genetically modified CHO cells, have a glycan structure closer to the natural human glycoproteins. However, the document US 5,047,335 does not describe what combination of glycosyltransferases would be necessary to obtain the recombinant ECG with biological activity.

The documents US 6.103.501, US 6.238.890 and US 6.987.172 describe artificial variants of single chain gonadotropins that are naturally found in the form of heterodimers, such as, for example, CG, TSH, LH and FSH, and which can provide effects or functions, or can behave as agonists or antagonists of the native hormones. Also, in this case, the biological activity of human CG is easily distinguished from the human LH activity because, unlike what occurs in horses, the β chains of these two gonadotropins are encoded by distinct genes in the humans. In addition, these documents do not describe what combination of glycosyltransferases would be necessary for obtaining the recombinant eCG biological activity.

The document US 2010/120677 describes methods of producing biologically active recombinant eFSH analogs and methods for improve the reproduction in mammals using recombinant eFSH analogs.

The document CA 2183564 describes methods for enhancing fertility by reducing the activity or levels of hormones with LH activity, and methods for selecting antibodies to specific portions of certain proteins including LH and hCG, to reduce its biological activity.

The prior art mentioned above shows the continuous efforts directed to the development of new recombinant gonadotropins and processes to produce them with safety, quality and unlimited way, emphasizing, even more the need for new solutions and the importance of Research & Development in this field of activity.

There remains, therefore, a need for improvements in the recombinant eCG production process, so that this is produced in large quantities, and at the same time has the glycosylation profile required for its *in vivo* biological activity. Surprisingly, the applicant found that it is possible to combine high productivity of recombinant eCG and obtain a glycosylation profile similar to that found in wild-eCG, ensuring, thereby, that the recombinant eCG thus obtained possesses *in vivo* biological activities similar to the FSH and LH hormones. This technical effect is unexpected because until then it was unknown which is the heterologous expression system based on mammalian cells would be capable of promoting post-translational modifications like N-linked and O-linked glycosylation in large amounts of recombinant eCG, necessary to differentiate the biological activity of eCG from that of the eLH. As discussed in detail above, it is not possible to predict with accuracy which factors are associated with obtaining such glycosylation profile compatible with an activity of eCG-type, especially: 1) which heterologous expression system would mimics the combination of enzymes and factors found in the endometrial cup cells, which are cells that naturally produce specifically the eCG; or 2) what are the conditions for generating a heterologous system and what are the conditions of cultivation of heterologous system associated with obtaining such glycosylation profile. Therefore, from this knowledge, in the present invention it has been possible to improve methods for producing recombinant eCG, ensuring the production of large quantities of recombinant eCG presenting a glycosylation profile similar to that of commercially available chorionic gonadotropin and, consequently, also presenting biological activity similar to that of wild-eCG. Such recombinant chorionic gonadotropins are produced through a system biotechnologically prepared for high expression of these structures.

A cell containing the gene expression of transcripts via glycosylation pathway enzymes cited below allows to obtain reCG with biological activity in the target species: UDP-GlnNAc-2-epimerase, CMP-SA-synthetase, CMP-SA-transporter, β-galactosidase α-2,3-sialyltransferase 3 (ST3-GalTIII) β-galactosidase α 2,3-sialyltransferase 4 (ST3-GalTIV), β-galactosidase α 2,3 sialyltransferase 6 (ST3-GalTVI) Sialidase I, Sialidase II.

### Summary of the Invention

The present invention relates to a method of producing a recombinant equine chorionic gonadotropin, in which said reCG presents glycosylation profile similar to that of commercially available chorionic gonadotropin. Said process comprises the steps of:
(a) inserting a DNA sequence encoding an eCG in an expression vector including cDNA encoding the dihydrofolate reductase enzyme (DHFR);
(b) co-transfecting the vector of step (a) with a selection vector in cells of double negative expression for DHFR gene;
(c) selecting transfected cells resistant to antibiotic treatment to which they shall present resistance;
(d) gene amplification of the expression vector and selection of superproducing cells of reCG by treatment with increasing concentrations of the DHFR inhibitor;
(e) growing the superproducing cells with reCGαβ with certain gene expression profile for the transcripts of enzymes of the glycosylation pathways for expression of reCGαβ;
(f) purifying the obtained reCGαβ.

According to a second aspect of the present invention, a recombinant equine chorionic gonadotropin is provided, produced from the process of the present invention. As said reCG produced from a fused cDNA chain containing both sequences encoding equine chorionic gonadotropin (eCG) α and β chains or cDNA chains containing the sequences encoding the α and β chains separately.

In a third aspect, the present invention relates to a veterinary composition comprising:
(a) a veterinary effective amount of reCG produced according to the present invention;
(b) optionally additives; and
(c) a pharmaceutically acceptable carrier.

In a fourth aspect, the present invention relates to the use of reCG of the invention or composition of the invention comprising said reCG, in the manufacture of a medicine for the treatment of medical conditions related to reproduction and ovulation of mammals, as enhancer agent for *in vitro* fertilization; in the manufacture of kits for diagnosis of veterinary conditions and protocols related to ovulation in mammals; and supplement manufacturing for cell culture.

### Brief Description of the Figures

Figure 1 shows an illustrative scheme used for constructing fused DNA encoding reCG by PCR.
Figure 2 shows SEQ ID NO: 1 in FASTA format.
Figure 3 illustrates the expression of reCGβα by populations of CHO-DG44 cells transfected with the plasmid pNUØ-eCGβα along the gene amplification process of this plasmid by treatment with increasing doses of the chemotherapeutic agent MTX.
Figure 4 illustrates the kinetics of expression reCGβα by CHO population 2,5µM after adaptation to growth in the absence of MTX over three days in the presence of fetal calf serum (FCS, *fetal calf serum*) or its absence (SFM, *serum free media*).
Figure 5 shows the *in vitro* biological activity of eCG evaluated from the progesterone response secreted by MLTC-1 cells after treatment with increasing doses of Folligon or reCGβα produced in the presence (A) or absence (B) of fetal bovine serum.
Figure 6 shows the *in vivo* biological activity evaluated from the uterine response of prepubertal female rats between 23 and 25 days after treatment with increasing doses of Folligon or reCGβα produced in the absence of fetal bovine serum.
Figure 7 shows the linear regression related to the data obtained in the *in vivo* biological activity assay evaluated from the uterine response of prepubertal female rats between 23 and 25 days after treatment with increasing doses of Folligon or reCGβα produced in the absence of fetal bovine serum.
Figure 8 illustrates the *in vivo* biological activity of partially purified reCG by column HiTrap™ Con A from the uterine response of prepubertal female rats of 25 days after treatment with Folligon or reCGβα produced in the absence of fetal bovine serum and partially purified by column HiTrap™ Con A.
Figure 9 illustrates N-glycan structures obtained for the commercial Folligon after digestion performed with exoglycosidases combinations.
Figure 10 illustrates the putative structures of N-glycans obtained in enriched fractions in reCGβα.
Figure 11 illustrates the gene expression profile obtained for the transcripts of enzymes corresponding to various enzymes of glycosylation pathways in the cells cultured in the presence and absence of fetal bovine serum: UDP-GlnNAc-2-epimerase, CMP-AS-synthetase, CMP-SA-transporter, β-galactosidase α 2,3-sialyltransferase 3 (ST3-GalTIII), β-galactosidase α-2,3-sialyltransferase 4 (ST3-GalTIV), β-galactosidase, α-2,3 sialyltransferase 6 (GalTVI-ST3), Sialidase I, Sialidase II.

### Detailed Description of the Invention

The present invention relates to a production process of a recombinant equine chorionic gonadotropin (reCG), wherein said recombinant equine chorionic gonadotropin presents glycosylation profile similar to that of natural equine chorionic gonadotropin and presents the same *in vivo* activity level of FSH and LH hormones.

According to a first aspect of the invention, said process comprising the steps of:
(a) inserting a DNA sequence encoding an eCG in an expression vector including cDNA encoding the dihydrofolate reductase enzyme (DHFR);
(b) co-transfecting the vector of step (a) with a selection vector in cells of double negative expression for DHFR gene;
(c) selecting the transfected cells resistant to antibiotic treatment to which they shall presents resistance;
(d) gene amplification of the expression vector and selection of superproducing cells of reCG by treatment with increasing concentrations of the DHFR inhibitor;
(e) growing the superproducing cells with reCGαβ with certain gene expression profile of transcripts of enzymes of the glycosylation pathways for expression of reCGαβ;
(f) purifying the obtained reCGαβ.

The DNA sequence encoding the eCG, according to the present invention preferably comprises a cDNA chain fused SEQ ID NO. 1, both containing the coding sequences of the equine chorionic gonadotropin (eCG) chains α and β.
SEQ ID NO.1:

CGGAATTCGCCACCATGGAGACGCTCCAGGGGCTGCTGCTGTGGATGCT
GCTGAGTGTTGGCGGGGTCTGGGCATCCAGGGGGCCACTGCGGCCACTG
TGCCGGCCCATCAACGCCACTCTGGCTGCTGAGAAGGAGGCCTGCCCCA

TCTGCATCACCTTCACCACCAGCATCTGTGCCGGCTACTGCCCCAGCATG
GTGCGGGTGATGCCAGCTGCCCTGCCGGCCATTCCCCAGCCAGTGTGCA
CCTACCGTGAGCTGCGCTTTGCTTCCATCCGGCTCCCCGGCTGCCCGCCT
GGTGTGGACCCCATGGTCTCCTTCCCCGTGGCCCTCAGTTGTCACTGCGG
GCCCTGCCAGATCAAGACCACTGACTGCGGGGTTTTCAGAGACCAGCCCT
TGGCCTGTGCCCCCCAGGCCTCCTCTTCCTCTAAGGATCCCCCATCCCAA
CCTCTCACATCCACATCCACCCCAACTCCTGGGGCCAGCAGACGTTCCTC
TCATCCCCTCCCAATAAAGACTTCTTTTCCTGATGGAGAGTTTACAACGCA
GGATTGCCCAGAATGCAAGCTAAGGAAAACAAGTACTTCTTCAAACTGG
GCGTCCCGATTTACCAGTGTAAGGGCTGCTGCTTCTCCAGAGCGTACCCC
ACTCCAGCAAGGTCCAGGAAGACAATGTTGGTCCCAAAGAACATCACCTC
AGAATCCACATGCTGTGTGGCCAAAGCATTTATCAGGGTCACAGTGATGG
GAAACATCAAGTTGGAGAACCACACCCAGTGCTATTGCAGCACTTGCTATC
ACCACAAGATTTAAGCGGCCGCGC

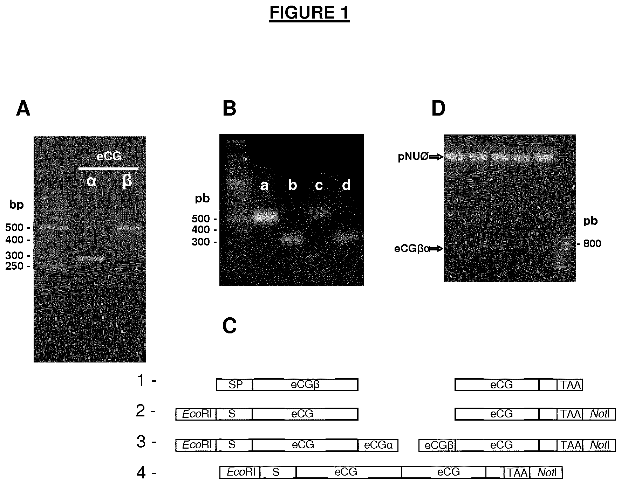

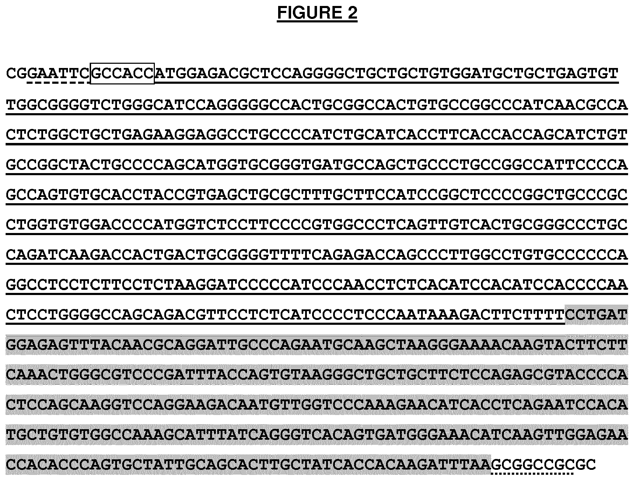

The sequence of gene encoding the reCG was designed to contain, preferably, sequences encoding both units of eCG, α and β, in a way they were expressed in a fused form, as can be seen in Figure 1, and in Figure 2. Figure 2 shows SEQ ID NO: 1 in FASTA format, chromatograms obtained after submitting the sequence of one of the bacterial clones containing the construct pNUØeCGβα to automated sequencing, using the Sanger method.

In Figure 2, the dashed sequence
GAATTC
represents the site for the restriction enzyme *Eco*RI; the contoured sequence
GCCACC
represents the Kozak fragment; the underlined sequence 
ATGGAGACGCTCCAGGGGCTGCTGCTGTGGATGCTGCTGAGTGTTGGCG
GGGTCTGGGCATCCAGGGGGCCACTGCGGCCACTGTGCCGGCCCATCAA
CGCCACTCTGGCTGCTGAGAAGGAGGCCTGCCCCATCTGCATCACCTTCA
CCACCAGCATCTGTGCCGGCTACTGCCCCAGCATGGTGCGGGTGATGCC
AGCTGCCCTGCCGGCCATTCCCCAGCCAGTGTGCACCTACCGTGAGCTG
CGCTTTGCTTCCATCCGGCTCCCCGGCTGCCCGCCTGGTGTGGACCCCAT
GGTCTCCTTCCCCGTGGCCCTCAGTTGTCACTGCGGGCCCTGCCAGATCA
AGACCACTGACTGCGGGGTTTTCAGAGACCAGCCCTTGGCCTGTGCCCCC
CAGGCCTCCTCTTCCTCTAAGGATCCCCCATCCCAACCTCTCACATCCACA
TCCACCCCAACTCCTGGGGCCAGCAGACGTTCCTCTCATCCCCTCCCAAT
AAAGACTTCTTTT
 represents β chain without the *stop codon*; highlighted sequence 
CCTGATGGAGAGTTTACAACGCAGGATTGCCCAGAATGCAAGCTAAGGGA
AAACAAGTACTTCTTCAAACTGGGCGTCCCGATTTACCAGTGTAAGGGCTG
CTGCTTCTCCAGAGCGTACCCCACTCCAGCAAGGTCCAGGAAGACAATGT
TGGTCCCAAAGAACATCACCTCAGAATCCACATGCTGTGTGGCCAAAGCAT
TTATCAGGGTCACAGTGATGGGAAACATCAAGTTGGAGAACCACACCCAG
TGCTATTGCAGCACTTGCTATCACCACAAGATTTAA
 represents α chain without the signal peptide; and the dotted sequence
GCGGCCGC
represents the site for the *Not*I restriction enzyme. The total length of the construction is 822pb.

The *primers* used for amplification of cDNAs sequences were designed from the complete coding sequences of each subunit of eCG contained in the NCBI database (http://www.ncbi.nlm.nih.gov; *National Center for Biotechnology Information*, U. S. NLM - U. S. National Library of Medicine, Bethesda, EUA (NM access number α: 001099763.1, β: 001490342.2). A computer program for the design of oligonucleotides has not been used.

Amplification of the coding chains may be accomplished by PCR technique (*Polymerase Chain Reaction*) and the obtained products are separated and purified by commonly known techniques employed in the prior art. Preferably, the obtained *amplicons* are detected on an agarose gel, isolated and purified through *Illustra GFX PCR DNA and Gel Band Purification Kit* (GE Healtcare, Carlsbad, CA, USA).

In another aspect of the invention sequences of the encoding gene comprising only one of the sequences encoding the α or β units of eCG can be designed.

More specifically, the construction of the fused gene encoding reCG occurs from a sequence of PCR reactions, divided into three stages, with the purified product. Between each *round* of PCR, the obtained product was purified on agarose gel.

The first *round* of PCR is responsible for adding restriction sites at the end of the eCGα sequence and at the beginning of eCGβ sequence. In the second *round,* complementary gene fragments are added to the counter-subunit sequence. A complementary fragment was added to both eCGα and eCGβ chains, allowing the binding of subunits through these fragments. The third *round* is responsible for binding the mutated eCGα and eCGβ chains in a single chain, eCGβα. The obtained *amplicons* were detected on agarose gel and purified with *Illustra GFX PCR DNA and Gel Band Purification Kit* (GE Healtcare, Carlsbad, CA, USA).

After purification of the fused gene on agarose gel, the gene was digested with restriction enzymes *Eco*RI and *Not*I and subcloned into a plasmid vector.

The gene can be cloned into any plasmid vector containing one or more strong promoters, including the chicken β-actin promoter and cytomegalovirus promoter, multiple site of bacterial cloning, resistance sequence to antibiotic for bacterial cloning, recombination sites, and cDNA encoding of the dihydrofolate reductase (DHFR) enzyme downstream of an internal entry site for ribosomes (IRES - *international ribosomal entry site*). Preferably, the plasmid expression vector suitable for the invention is pNUØ vector, constructed by the applicant itself.

The pNUØ vector is derived from the pUC18 vector (MESSING E VIEIRA, The pUC plasmids, an M13mp7-derived system for insertion mutagenesis and sequencing with synthetic universal primers. Gene. 1982 Oct;19(3):259-68) and it has an MCS cloning cassette containing several sites for restriction enzymes under the control of a hybrid promoter containing *enhancer* element of cytomegalovirus and chicken β-actin promoter as well as the cDNA encoding the dihydrofolate reductase (DHFR) enzyme downstream of an internal entry site for ribosomes.

This structure enables the concomitant expression of the insert cloned into the MCS, in this case the eCGβα, and DHFR, aiming at genetic complementation of double negative CHO cells for the DHFR gene, which, in turn, allows selection of cells expressing high amounts of the product of interest after gene amplification, carried out by treatment with increasing levels of DHFR inhibitors.

The constructed plasmid was then co-transfected into cells of interest along with a selection vector for the generation of cell clones stably expressing the fused protein eCGβα.

The insertion of the plasmid in the cell of interest (co-transfection) can be carried out by various techniques commonly employed in the state of art. Among them, several types of transfection, such as, for example, by means of cationic molecules, calcium-mediated transfection, PEI, electroporation, nucleofection since the transfectability rate is maintained in the same order of magnitude.

Preferably, the co-transfection is carried out by lipofection with selection vector. Preferably, the selection vector provides resistance to the antibiotic hygromycin B. More preferably, the appropriate selection vector is the pX343vector (ARMELIN et al., Functional role for c-myc in mitogenic response to platelet-derived growth factor. Nature. 1984 Aug 23-29;310(5979):655-60).

Co-transfection of construction pNUØeCGβα in the cells of interest with the pX343 vector was performed in a ratio of pX343 vector to construction pNUØeCGβα ranging from 1: 1 to 1: 5000. Preferably, co-transfection was performed in a ratio of 1:120. Therefore, few copies of the resistance vector are necessary to offer resistance to the transfected cells when the process of the present invention is employed.

Further, the transfected cells, which were selected because they are hygromycin-resistant due to the presence of at least one copy of pX343 vector, have the advantage of containing a much higher amount of the construction of interest and hence a high expression of reCG protein.

As the eCG is a complex molecule, presenting about 45% of its molecular weight consisting of post-translational modifications (glycosylation), it is necessary to use a robust expression system which allows the production of recombinant eCG as similar as possible compared to the wild-type eCG. The heterologous expression system according to the present invention was chosen to produce reCG because it allows the correct processing of newly synthesized polypeptide chains, folding and assembly of its α and β subunits, plus the addition of complex-type glycosylated chains, which is essential for the produced recombinant eCG has biological activity. However, there is great variability in glycosylation patterns among different cells of mammals due to fact that the enzymatic machinery of adding oligosaccharides to proteins differ among cell lines, generating glycoforms with different degrees and types of glycosylation, and distinct biological activities.

The cells used for the expression of reCG are mammalian line cells or insect cells, or yet any cell line that has certain gene expression profile for the transcripts of enzymes of the glycosylation pathways. Therefore, any other cell line that is capable of adding post-translational modifications similar to those obtained for the reCG profile defined for the present invention, with the major presence of peaks corresponding to di- or tri-galactosylated N-glycans being further mono-, di-, or tri-sialyated, added by UDP-GlnNAc-2-epimerase, CMP-SA-sintetase, CMP-SA-transporter, β-galactosidase α 2,3-sialyltransferase 3 (ST3-GalTIII), β-galactosidase α 2,3-sialyltransferase 4 (ST3-GalTIV), β-galactosidase α 2,3-sialyltransferase 6 (ST3-GalTVI), Sialidase I, Sialidase II enzymes, and that has the same behavior when *in vitro* cultured, for example, replication ability, ability of protein expression, simple transfectability, among others, can be used.

Preferably, the mammalian line cells were chosen; most preferably, CHO line cells or derived therefrom. Even more preferably, CHO-DG44-DHFR^{-/-} line is used. However, other cell types, such as CHO line cells, BHK, 293, Vero or derivatives thereof, are suitable for the invention and can replace the CHO-DG44-DHFR^{-/-} line cells.

The step of selection of resistant transfected cells was carried out by treating the transfected cells with the antibiotic to which they should present resistance for two weeks. Preferably, the CHO-DG44 cells, according to the invention, were treated with between 10 mg/mL to 2,000 ug/mL of hygromycin B (Life Technologies™).

After treatment with hygromycin B, the surviving cells were treated with increasing concentrations of the DHFR inhibitors in nucleoside-free culture medium for gene amplification of the plasmid and selection of superproducing cells of reCG.

Various DHFR inhibitors are known from the state of art and may be suitable for use in the invention. Preferably, the DHFR inhibitor suitable for the invention is Methotrexate (MTX).

Cells deficient for producing the DHFR enzyme, as CHO-DG44 DHFR -/- cells, preferably used in the present invention are suitable because they cannot survive when grown in the absence of nucleosides, as this enzyme catalyses the reduction dihydrofolate to tetrahydrofolate. Thus, when these cells are transfected with constructs? obtained from the vector of the invention having a gene sequence corresponding to the DHFR enzyme, they become capable of growing in the absence of nucleosides.

The cells were subjected to an initial concentration of DHFR inhibitor and cultured for about 2 or 3 weeks, changing the medium every 48 hours for removal of dead cells. When 90% confluence was reached, the concentration of the DHFR inhibitor was increased and the cycle was repeated, always increasing concentrations of the DHFR inhibitor.

The superproducing cells of reCG can be cultured in batches of production in monolayers or in suspension. Different reservoirs may be employed, such as cultivation bottles of different sizes, plates, T-flasks, *spinners*, disposable culture bag (*bags*) and other types of bioreactors with rounded blades or rods, among other configurations.

The culture media employed may vary since they are able to meet the metabolic needs of the cells. Furthermore, superproducing cells of reCG can be cultured in the presence or absence of fetal calf serum (FCS). The ratio of fetal bovine serum may be adapted according to the culture conditions to maintain cell viability and protein expression levels (from 1 to 100 mg / mL) within the same order of magnitude of the original pattern. Preferably, the cells of the invention are cultured in medium containing about 2 to 10% FCS. More preferably, the cells of the invention are cultured in medium containing 7% FCS.

Chemically defined media or serum- and protein-free containing FCS substitutes such as peptide factors of cell growth and proliferation of various types, albumin, insulin, among others; or fetal or adult serum of other species and supplemented with precursors used in the glycosylation pathways are also suitable for use in the production of reCG. The culture should occur at defined environmental conditions such as culture temperature between 25 and 40°C, relative ratio of CO₂ between 1 and 10%, and pH between 5,0 and 8,0.

When the culture is conducted in *spinners*, *bags*, and bioreactors the employed rotation can vary between 10 and 200 rpm.

To achieve greater mass and purity of the protein of interest, the obtained product can be partially purified using known techniques for protein purification, wherein the conditioned culture media containing collected reCGβα are subjected to at least one of the following techniques: precipitation of proteins using solvents such as ammonium sulfate; ultrafiltration; tangential flow filtration; or two different types of chromatography, ion exchange chromatography and affinity chromatography.

According to a second aspect of the present invention, there is provided a recombinant equine chorionic gonadotropin, produced from the process of the present invention. Unlike commercially available options, recombinant equine chorionic gonadotropin of the invention is produced by biotechnological mechanisms on an industrial scale by offering an alternative option to those obtained from equine plasma and involve bioethical factors. Furthermore, reCG of the invention does not face the problems found in the prior art related to the lack of source's origin, advantageously constituting a new source of uninterrupted supply of reCG.

When producing one reCG with glycosylation pattern similar to the one of commercially available chorionic gonadotropin, and to present the same level of *in vivo* activity of FSH and LH hormones, there is provided an obtained product with at least a similar biological activity. This feature is not presented by recombinant chorionic gonadotropin known in the prior art, which even had *in vivo* biological activity.

In a third aspect, the present invention relates to a veterinary composition comprising:
a) a veterinary effective amount of reCG produced according to the present invention;
b) optionally additives; and
c) a pharmaceutically acceptable carrier.

The composition of the present invention may comprise, besides the reCG of the invention, adjuvants, preservatives, diluents, emulsifiers, stabilizers, and other pharmaceutically acceptable ingredients which are employed in gonadotrophin preparations for animal use.

In a fourth aspect, the present invention relates to the use of (a) reCG or (b) veterinary composition of the invention comprising said reCG, in the manufacture of a medicine for the treatment of veterinary conditions related to reproduction and ovulation of mammalian animals, such as enhancer agent of the *in vitro* fertilization; in the manufacture of diagnostic kits of veterinary conditions and protocols related to ovulation of mammals; and in the supplement manufacturing for cell culture.

### EXAMPLES

### Example 1: Extraction of RNA and cDNA synthesis from an equine pituitary

One pituitary gland was obtained through necropsy of a horse (*Equus caballus*), male, arabic half-blood race, 19 years old, and immediately wrapped in RNAholder (Bioagency® Biotechnology) at 4°C, until macerated in liquid nitrogen using a pestle and crucible. Total RNA was extracted from this mash pituitary using *Illustra RNAspin Mini RNA Kit* (GE Healthcare®, Buckinghamshire, England), following the manufacturer's recommendations. The concentration and purity of obtained RNA were determined by quantification in a nano spectrophotometer (ND-1000 *Spectrophotometer*, NanoDrop Technologies Inc., Wilmington, USA) and the reading of the absorbance ratio of Abs260/Abs280 and Abs260/Abs230 nm.

An aliquot of 1,0µg of total RNA from the equine pituitary was used as template for a reverse transcription reaction using the enzyme kit *ImProm™-II RT* (Promega Corporation, Madison, USA); *Super Script II* (Invitrogen), being the reactions carried out according to the manufacturer's recommendations. Finally, the samples were diluted 3 times in deionized water, to obtain a final volume of 60 µL and stored at -80ºC.

### Example 2: Amplification of the chains encoding eCG α and β, from this cDNA library, by PCR

The *primers* used for amplification of cDNA sequences were designed based on the complete coding sequences of each of the two eCG subunits contained in the NCBI database (http://www.ncbi.nlm.nih.gov; *National Center for Biotechnology Information*, U. S. NLM - U. S. National Library of Medicine, Bethesda, EUA (NM_access number α: 001099763.1, β: 001490342.2) and synthesized by Thermo Fisher Scientific Inc. company, Waltham, USA™/Thermo Fisher Scientific Inc. The lyophilized *primers* were reconstituted in buffer solution 10 mM Tris-CI (pH 8,0) to provide a stock solution 100 µM and use solutions 10 µM. The sequences of the *primers* used in the amplification of such chains and their intermediates obtained in steps of the Genetic Engineering are set forth in Table 1.

**Table 1. Sequences of the primers for amplification of the sequences encoding reCGα and reCGβ and genetic engineering for generating fused sequence eCGβα**

| **Oligonucleotides** | **Sequences (5'-3')** |
|---|---|
| 1 - eCGb F | 5'-ATGGAGACGCTCCAGGGGCT- 3' |
| 2 - eCGb R | 5'-AGAAGTCTTTATTGGGAGGGGATGAGAG- 3' |
| 3 - eCGa F | 5'-TTTCCTGATGGAGAGTTTACAACGCAG- 3' |
| 4 - eCGa R | 5'-TTAAATCTTGTGGTGATAGCACGTGCTG-3'. |
| 5 - Mut eCGa R *Not*I | 5'-GC**GCGGCCGC**TTAAATCTTGTTGTGGTG-3' |
| 6 - Mut eCGb F *Eco*RI | 5'-CG**GAATTC**GCCACCATGGAGACGCTC-3' |
| 7 - union F | 5´-CCCAATAAAGACTTCTTTTCCTGATGGAGAG- 3’ |
| 8 - union R | 5’- CTCTCCATCAGGAAAAGAAGTCTTTATTGGG-3’. |

| | |
|---|---|
| Bold: restriction sites for *Eco*RI (*forward primer*) and *Not*I (*reverse primer*) enzymes; Underline: consensus sequence of Kozak. | |

The total cDNA of equine pituitary tissue was used for amplification of the encoding sequences of both eCG subunits. A single chain encoding both eCG subunits was created (JABLONKA-SHARIFF et al., Expression and bioactivity of a single chain recombinant equine luteinizing hormone (reLH). Theriogenology. 2007 Jan 15;67(2):311-20), so that the subunits were expressed in a fused form. The *primers* (1) and (2) were used to amplify the sequence encoding eCGβ without the stop codon, and sequence encoding eCGα was amplified without the signal peptide, using (3) and (4) *primers.*

For amplification, the polymerase chain reactions (PCR) were performed using the enzyme *High Fidelity Platinum Taq DNA Polymerase* (Thermo Fisher Scientific Inc., Waltham, USA™). To 100 ng of equine pituitary cDNA samples were added to 1X buffer *High Fidelity PCR Buffer* (Thermo Fisher Scientific Inc., Waltham, USA), 0,2 mM of dNTPs (Thermo Fisher Scientific Inc., Waltham, USA), 2 mM of MgSO₄ (Thermo Fisher Scientific Inc., Waltham, USA), 400 nM of *Forward primer* (Thermo Fisher Scientific Inc., Waltham, USA), 400 nM of *Reverse primer* (Thermo Fisher Scientific Inc., Waltham, USA), 1,0 U of DNA polymerase enzyme described above and deionized water to complete 50,0 µL of the reaction volume. The reaction was conducted under the following conditions: 94ºC for 1 minute; 35 cycles of: 94ºC for 30 seconds, 55ºC (eCGα) or 58ºC (eCGβ) for 30 seconds, and 68ºC for 1 minute; final extension at 68ºC for 2 minutes.

The *amplicons* were detected through 2,0% agarose gels stained with 0,5 µg/mL of ethidium bromide, and the product of the expected size was isolated with a scalpel and purified by *GFX DNA and Band Purification Kit* (GE Healtcare, Carlsbad, CA, USA) according to manufacturer's recommendations.

### Example 3: Obtaining a fused cDNA chain containing both sequences encoding for eCG (eCGβα)

The purified products were used as templates for the obtaining of eCGβα sequence through three steps of subsequent mutagenic PCR reactions, as outlined in Figure 1, in which **(A)** amplifying the sequence encoding eCGα without the signal peptide (α: 288 bp) and the sequence encoding eCGβ without *stop codon* (β: 510 bp)/**(B)** intermediate a-b and c-d constructions, obtained respectively on the first and second *round* of mutagenic PCRs/**(C)** Schematic diagram representing the steps in Genetic Engineering performed to obtain the final insert eCGβα - (1): representation of Figure 1A; (2): representation of Figure 1B (a and b); (3): representation of Figure 1B (c and d); (4): representation of the final product eCGβα/**(D)** Digestion of pNUØ-eCGα plasmid with the restriction enzymes *Eco*RI and *Not*I, where it is possible verified the pNUØ vector (7,6 kbp) and the insert (822 bp) present in different bacterial clones (SP: signal peptide. TAA:*stop codon*, and *Eco*RI and *Not*I: site for *Eco*RI and a *Not*I restriction enzymes).

The first step of PCR reaction (Figure 1A) adds restriction sites at the end of the eCGα sequence (*Not*I) using *primers* (3) and (5), and at the beginning of the sequence eCGβ (*Eco*RI), using *primers* (2) and (6). PCR reactions were performed using the enzyme *Long Range* (Thermo Fisher Scientific Inc., Waltham, USA) in which to 100 ng of plasmid preparations containing eCGβ or eCGα sequences were added 1 X buffer *High Fidelity PCR Buffer* (Thermo Fisher Scientific Inc., Waltham, USA), 0.2 mM of dNTPs (Thermo Fisher Scientific Inc., Waltham, USA), 2 mM of MgSO₄ (Thermo Fisher Scientific Inc., Waltham, USA), 400nM of *forward primer* (Thermo Fisher Scientific Inc., Waltham, USA), 400 nM of *reverse primer* (Thermo Fisher Scientific Inc., Waltham, USA), 1,0 U of DNA polymerase enzyme described above and deionized water to complete 50.0 µL of reaction volume. The reaction was conducted under the following conditions: 94ºC for 1 minute; 35 cycles of: 94ºC for 30 seconds, 55ºC (eCGα) or 58ºC (eCGβ) for 30 seconds and 68ºC for 1 minute; final extension at 68ºC for 2 minutes. The obtained products were fractionated on agarose gel and then in a similar procedure to that described in the paragraph above, purified of agarose gel to serve as templates in the next step of mutagenic reactions.

In the second step of PCR reaction, Figure 1B, gene fragments were added to both amplicons complementary to the counter-subunit sequence, so that both subunits could be joined through these fragments. Initial complementary fragment of eCGα chain was added at the end of eCGβ chain modified in a reaction that used the *primers* (6) and (7), whereas the complementary final fragment of eCGβ chain was added to the start of eCGα chain and the start of eCGβ sequence (*Eco*RI), using the *primers* (5) and (8). For this second step of mutagenic PCR reactions, the same reagents and concentrations of the first step were used under the following cycling conditions: 94ºC for 1 minute; 35 cycles of 94ºC for 30 seconds, 62ºC for 30 seconds, and 68ºC for 1 minute; final extension at 68ºC for 2 minutes. Again, the products of these reactions were purified from agarose gel using the *Illustra GFX PCR DNA and Gel Band Purification Kit* and used as *template* for the last step of mutations.

The third step of mutagenic PCRs, Figure 1B and 1C, has as objective to join the two previously mutated chains into a single chain, eCGβα. The purified *templates* from the previous step were added in a PCR reaction performed with *primers* (5) and (6) and the same reagents and concentrations described under the following conditions: 94ºC for 1 minute; 35 cycles of 94ºC for 30 seconds, 65ºC for 30 seconds, and 68ºC for 1 minute; final extension at 68ºC for 2 minutes.

The *amplicons* were detected using 2.0% agarose gels stained with 2,0% of ethidium bromide, and the product of the expected size was isolated with a scalpel and purified by *Illustra GFX PCR DNA and Gel Band Purification Kit* (GE Healthcare, Carlsbad, CA, USA). Next, they were cloned in pGEM® vector using reagents supplied by *Promega pGEM®-T Easy System* (Promega Corporation, Madison, USA) in *Escherichia coli*, XL-1 Blue strain (genotype: recA1 endA1 gyrA96 thi-1 hsdR17 supE44 relA1 lac [F' proAB laclqZΔM15 Tn10 (Tetr)], Agilent Technologies, Santa Clara, USA).

The sequences of interest were checked in the bacterial clones by automated sequencing (*BigDye Sequencing Kit*, Thermo Fisher Scientific Inc., Waltham, USA, on sequencer Model ABl3700 l377 (Perkin Elmer)). The sequences obtained in sequencing were analyzed and aligned through the PhredPhrap programs (EWING, HILLIER et al., Base-calling of automated sequencer traces using Phred I. Accuracy assessment. Genome Res. 1998 Mar;8(3):175-85) e BLAST (http://www.ncbi.nlm.nih.gov/BLAST/), respectively.

Bacterial clones were then expanded in LB lysogenic medium (Becton, Dickinson and Company) and stored at -80ºC in freezing medium with 15% of glycerol.

### Example 4: Cloning of the eCGβα in the expression vector for mammalian cells

The pGEM®-eCGβα plasmid was isolated from the bacterial clone selected through *GeneJET Plasmid Miniprep Kit* (Thermo Fisher Scientific Inc., Waltham, USA.), digested with *Eco*RI and Not*I* restriction enzymes (Thermo Fisher Scientific Inc.). The digested insert was purified from agarose gel, and then subcloned into a specially constructed plasmid vector called pNUØ by our group.

For the subcloning, the vector was previously linearized by action of *Eco*RI and *Not*I enzymes, generating 5' and 3' cohesive ends and complementary to the eCGβα insert, using the molar ratio of insert: vector of 3:1 (M = 3 x MV x TI/TV, where MI = insert mass, MV = vector mass, TI = insert size, and TV = vector size). For the binding reaction of DNA fragments, i the enzyme T4 DNA ligase was used (New England Biolabs® Inc.) according to the manufacturer's recommendation. The reaction took place at 16ºC for 18h and gave rise to called pNUØ-eCGβα vector. The obtainment of positive bacterial clones of *Escherichia coli*, XL-1 Blue strain, containing this vector were kept in glycerinated solutions at -80°C, as described above. Plasmid preparations in mean scale of a positive clone were obtained through *QIAGEN Plasmid Midi Kit* (QIAGEN), following the manufacturer's guidelines, the concentration of the samples being determined by quantification in a nanospectrophotometer at 260 nm.

### Example 5: Transfection of the construction of interest in CHO-DG44 cells by lipofection

Cells of CHO-DG44 DHFR^{-/-} lines, of the Chinese Hamster Ovary were purchased as a donation and cultured in HamF12 medium (Thermo Fisher Scientific Inc., Waltham, USA™) supplemented with 10% (v/v) of FCS (Vitrocell Embriolife, Campinas, SP, Brasil) in adherent culture. The cells were always incubated in humid atmosphere at 37ºC and 2% of CO₂/98% of air and maintained in T-flasks or treated culture plates, suitable for I adherent cells culture (Thermo Fisher Scientific Inc.; Becton, Dickinson and Company, Corning Inc., Corning, USA; or TPP Techno Plastic Products, Trasadingen, Switzerland). The culture medium was changed every two or three days. Upon reaching confluence greater than or equal to 80%, the cells were subcultured, and washed with PBSA followed by addition of 0.1% of trypsin solution and inactivation of the same with culture medium containing FCS. The stock of cells was maintained freezing in culture medium supplemented with 10% of DMSO (Sigma-Aldrich Co., St. Louis, MO, USA) and storing in liquid nitrogen.

To generate cell clones stably expressing the eCGβα fused protein, CHO-DG44 cells cultured on plates of 60 mm in diameter (P60) were co-transfected with pNUØ-eCGβα construction and with the pX343 selection vector, which confers resistance to hygromycin B antibiotic, using a DNA mass ratio of 120:1 (from 8µg of the plasmid of interest to 66,7ng of the hygromycin-resistant plasmid) as previously described (ARMELIN et al., Functional role for c-myc in mitogenic response to platelet-derived growth factor. Nature. 1984 Aug 23-29;310(5979):655-60). The plasmids were first mixed with 16µL of *FuGENE HD Transfection Reagent* transfection reagent (Hoffmann-La Roche Ltd.) in culture medium in the absence of FCS for the formation of DNA-liposome complexes. Cells were incubated with the complex and the culture medium was exchanged after 24 hours.

### Example 6: Selection of a mixed population of transfected CHO-DG44 cells

48 hours after transfection, the CHO-DG44 cells began to be treated with 200 µg/mL of hygromycin B (Life Tehnologies™) for selection of resistant cells within the transfected population, for two weeks, until the selection of a resistant subpopulation.

The surviving cell to treatment with hygromycin must have integrated at least one copy of the resistance plasmid (in this case, pX343), therefore must have a much higher number of copies of the plasmid of interest (pNUØ)-eCGβα.

### Example 7: Gene amplification of pNU-Ø- eCGβα construction in CHO-DG44 cells through treatment with increasing doses of methotrexate (MTX)

Gene amplification took place through treatment with methotrexate, started with 30 nM of this drug added to the culture medium without nucleosides and 7% of dialyzed FCS (dFCS).

The cells were cultured for about two weeks, with medium exchange every 48 hours for removal of dead cells until the culture reached 90% of confluence, when then, it was increased MTX concentration. The treatment continued for about six months, with gradual increase of MTX concentration each time the culture reached 90% of confluence, each phase of treatment lasted two to three weeks.

### Example 8: Analysis of reCG expression in culture media conditioned by specific ELISA

10⁶ cells from populations of superproducing cells of reCGβα were plated in 1 mL of specific culture medium in the presence of dFCS in six well trays (B6). The plating was performed in duplicate, and the trays were maintained in culture conditions described in Example 5. 24h after plating, the culture medium was discarded, adherent cultures were washed with PBSA and received fresh culture medium. The medium of each clone/population was collected after 48h of conditioning and used for reCGβα dosage through a specific ELISA (PMSG ELISA - ALPCO Diagnostics) following the manual provided by the manufacturer. Some dilutions were performed of the collected conditioned media (1:10, 1:25, 1:50 and 1:100), in order to obtain values of reCGβα within calibration curve of kit. After the procedure, the absorbance values were determined at 460nm by reading on a plate spectrophotometer (SpectraMax M2 - Molecular Devices). A standard curve, provided by the kit, was used as reference for a logistic analysis of 4-parameters, performed by GraphPad Prisma 5.00 software for Windows (GraphPad Software, San Diego California USA, www.graphpad.com). The values obtained for each population were considered in the selection of a candidate for the continuation of the experiments relating to this project.

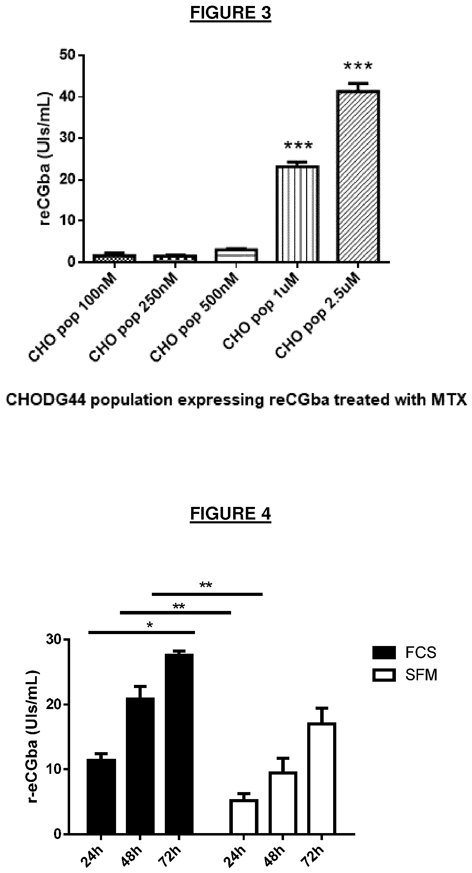

The level of expression of the reCGβα in the culture medium throughout the treatment with increasing doses of MTX can be seen in Figure 3. Figure 3 shows in its abscissa, the relative values of the populations of CHO-DG44 cells superexpressing reCGβα to which they are resistant to increasing doses of methotrexate (MTX); and its ordinate, values related to concentration of reCGβα (Uls/mL) present in the conditioned culture medium during 48 hours per cell population.

By means of the graph, it is possible to check that the expression of reCGβα maintains at similar levels up to the treatment with 500nM of MTX, increasing dramatically to 23.1 IUs and 41.3UIs of reCGβα being expressed in transfected CHO-DG44 cell populations subjected to growth in the presence of 1 µM and 2,5µM of MTX, respectively.

CHO-DG44 population with greater reCGβα expression was subjected to a dynamic of reCGβα expression in which 10⁶ cells of these cells were plated in B6 trays in the same manner as described in the above procedure, for 10 additional passages, with the addition of culture medium containing or not FCS (fetal bovine serum). Conditioned media were collected 24, 48 and 72 hours after the exchange, diluted (1:10, 1:25 and 1:50) and subjected to ELISA for PMSG, exactly as performed in the previous procedure. The level of reCGβα expression over time was determined again by a logistic analysis of 4-parameters, performed by GraphPad Prism 5.0 software.

Figure 4 illustrates the kinetics of reCGβα expression by 2,5 µM of CHO population after adaptation to growth in the absence of MTX over three days in the presence or absence of fetal bovine serum. The graph shows on its abscissa the values related to the points (24 h, 48 h or 72 h) for determining the concentration of reCGβα present in conditioned medium with or without FCS (*fetal calf serum*) and its ordinate values of the concentration of reCGβα (Uls/mL) present in the culture media conditioned 48 hours for each condition, the FCS (*fetal calf serum*) is the conditioned culture medium in the presence of fetal bovine serum, and SFM (*serum free media*) is the conditioned culture medium in the absence of dFCS.

By means of data provided by Figures 3 and 4, the expression of reCGβα by the population resistant to 2,5µM of MTX decreased after 10 passages of removal of the selection pressure exerted by MTX. Taking the 48h point as comparison parameter, this decrease was in the 2X range, from ∼ 40 Uls/mL with MTX (Figure 3) to ∼ 20 Uls without MTX (Figure 4) when cells were cultured in the FCS condition, and in the 4X range (from 40 Uls with MTX to ∼ 10 Uls without MTX), when cells were cultured in the SFM condition. Comparing the level of expression of the protein of interest between the FCS and SFM conditions, it can be said that the FCS condition showed higher expression of reCGβα which can be determined by ELISA. A statistical analysis of the data, performed by Two-Way ANOVA (GraphPad Prism) followed by Bonferroni post-hoc test has showed statistical significance (p <0.05) for this phenomenon at 24h point, and a difference with more stringent significance (p <0.01) between the FCS and SFM conditions was observed in 48h and 72h points of this population.

### Example 9: Assays of in vitro biological activity using the MLTC-1 -responsive cell line

To determine the *in vitro* biological activity of reCGβα produced by superproducing cells, the MLTC-1 cell line was used (*Mouse Leydig Tumor Cell* - ATCC-CRL2065), obtained from the ATCC®, which superexpress gonadotropin receptors (hCG and hFSH), and a suitable protocol LEGARDINIER et al. (LEGARDINIER et al., Involvement of equine chorionic gonadotropin (eCG) carbohydrate side chains in its bioactivity; lessons from recombinant hormone expressed in insect cells. Reprod Nutr Dev. 2005 May- Jun;45(3):255-9). This cell is responsive to treatment with eCG producing and secreting progesterone in a dose-dependent manner.

10⁵ MLTC-1 cells were plated in 24-well trays using 500µL of RPMI culture medium (Gibco®) supplemented with 10% of FCS. When they reached 80% of confluence, these cultures were washed 2X with PBSA and FCS-deprived for 2 hours, then, to be treated by additional 2h with various dilutions of conditioned culture media containing increasing doses of reCGβα and commercial eCG (Folligon - Intervet®). After treatment, these culture media conditioned by MLTC-1 cells were collected and stored at -20°C until submitted to progesterone dosing by HPLC. The dosage can also be performed through ELISA kits.

Preparations were used containing increasing doses of reCGβα resulting from CHO-DG44 cells cultured under the conditions in the presence (FCS) or absence of (SFM) fetal bovine serum as the experimental conditions. As a negative control a culture medium conditioned by CHO-DG44 cells not producing the reCGβα was used and Folligon as the positive control, the commercial eCG (Intervet).

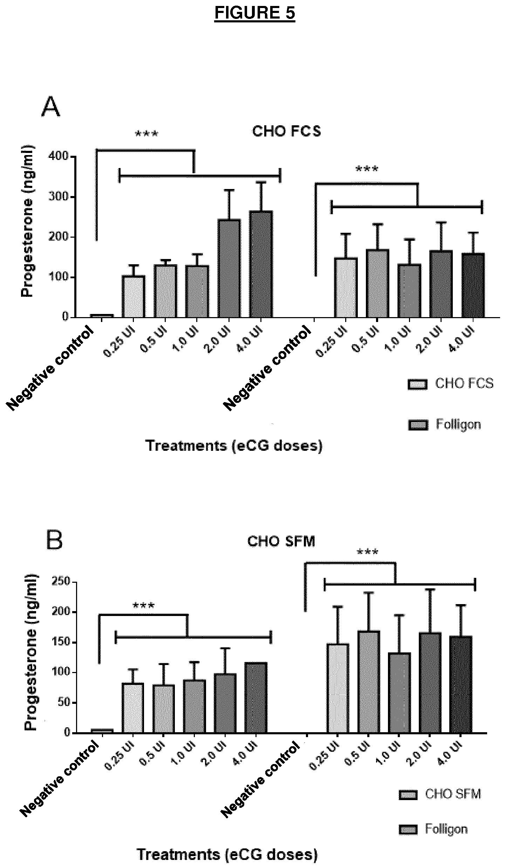

The *in vitro* biological activity was determined by the amount of progesterone contained in the samples, which was measured by high performance liquid chromatography technique performed by the company Nanocore Biotechnologia S/A. Two independent experiments were performed for each sample. It was performed the two-way ANOVA variance test, with Bonferroni post-hoc analysis, this analysis was performed in GraphPad Prism version 5.0. The *in vitro* biological activity obtained for each tested condition can be found in Figure 5.

Figure 5 shows the *in vitro* biological activity of eCG evaluated from the progesterone response secreted by MLTC-1 cells after treatment with increasing doses of Folligon or reCG of the present invention produced in the presence (A) or absence (B) of fetal bovine serum. The abscissa axis shows the values related to the performed treatments, consisting of increasing doses (0.25 IU; 0.5 IU; 1 IU, 2 IU, and 4IU) of reCG of the present invention or Folligon; and the ordinate axis shows values related to concentration of Progesterone (ng/mL) secreted in culture medium conditioned by MLTC-1 cells.

The results presented in Figure 5 show no difference in the response to the same dose of eCG resulting from different preparations (FCS or SFM, CHO-DG44 or Folligon). However, when the graphs are carefully observed, it is possible to note a tendency to a less robust response in serum-free medium conditions (SFM condition) when compared to the same doses in the presence of serum (FCS condition). This result is very positive, as indicates that the reCGβα produced by the transfected superproducing cells of reCG has *in vitro* biological activity similar to the commercially available product, Folligon, even when produced in the absence of fetal bovine serum. All points were statistically different (p <0.001) over the negative control.

### Example 10: Assays of in vivo biological activity using Cole & Erway assay

All experiments involving animals as well as methods used to creation and forms of maintenance and experimentation, followed the rules of the Ethics Committee on Animal Experiments (CEUA) from the Chemistry Institute of *USP.* The study was conducted through recommended assay by British Pharmacopoeia, described by Cole & Erway (Cole and Erway, 1941).

Sixty Female Sprague-Dawley rats of 25 days old, obtained in the Animal Experimentation Vivarium of Institute of Chemistry-Pharmaceutical Sciences Faculty of the University of Sao Paulo, were organized into groups of 5 to 10 animals per box, receiving water and food *at libitum* and maintained in periods of 12 h of light and 12 h of dark, at sustainted 22°C.

The animals were divided into the negative control group (which received PBSA or culture medium conditioned by non-producing cells of reCGβα), positive control group (receiving Folligon®), and experimental groups, the latter containing FCS conditions (media culture conditioned by superproducing cells of reCG of the present invention containing 7% of dFCS) or SFM (culture media conditioned by superproducing cells of reCGβα in the absence of FCS).

Each experimental group and the positive control comprises five points (2.5 IUs; 5.0 IUs; 10 IUs; 20 IUs, and 40 IUs) with five animals. The experiment was repeated three times. Each animal received a single dose of 200 µL, via subcutaneous injection, of a preparation filtered through filters with pore of 0,22 µm containing any of the above treatments.

Three days after injection, animals were euthanized using CO₂ camera, and ovaries and uterus of each animal were removed, dissected and weighed. The increase in weight of these organs, compared to negative controls, corresponds to the biological response of the animal to stimulation with eCG.

The final response to each dose was obtained from the arithmetic mean of the weight of the organs (ovaries or uterus) of five animals of each point. The comparison of response between the positive control doses (Folligon®) and reCG of the present invention has allowed us to draw a parallel between the strengths of the first and last. It was employed the One-way ANOVA variance test with Tukey post-hoc analysis, the analysis performed in program GraphPad Prism version 5.0. The data were also analyzed by linear regression.

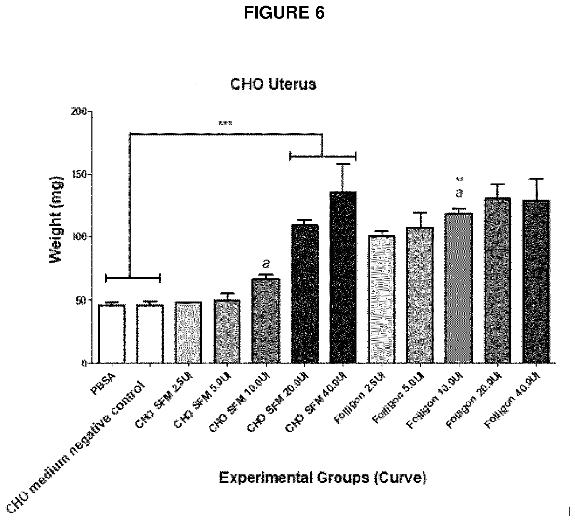

Figure 6 shows the *in vivo* biological activity evaluated as from the uterine response of prepubertal female rats between 23 and 25 days after treatment with increasing doses of Folligon or reCG of the present invention produced in the absence of fetal bovine serum. In the experiment illustrated in Figure 6, the increase in weight of the uterus of the treated animals was evaluated consisting in a direct response from animals to stimulation. The values related to performed treatments, consisting of increasing doses (2.5 IU; 5 IU, 10 IU, 20 IU, and 40 IU) of reCG of the present invention or Folligon, as well as negative controls PBSA (phosphate buffer in saline without Ca²⁺ and Mg²⁺) and CHO-DG44 cells not producing βα are represented on the abscissa axis, and values related to weight (mg) of the reproductive organs dissected on the fourth day after treatment are shown on the ordinate axis. The uterine response observed upon treatment with reCGβα produced by CHO-DG44 cells according to the present invention was, in fact, quite similar to that observed for Folligon.

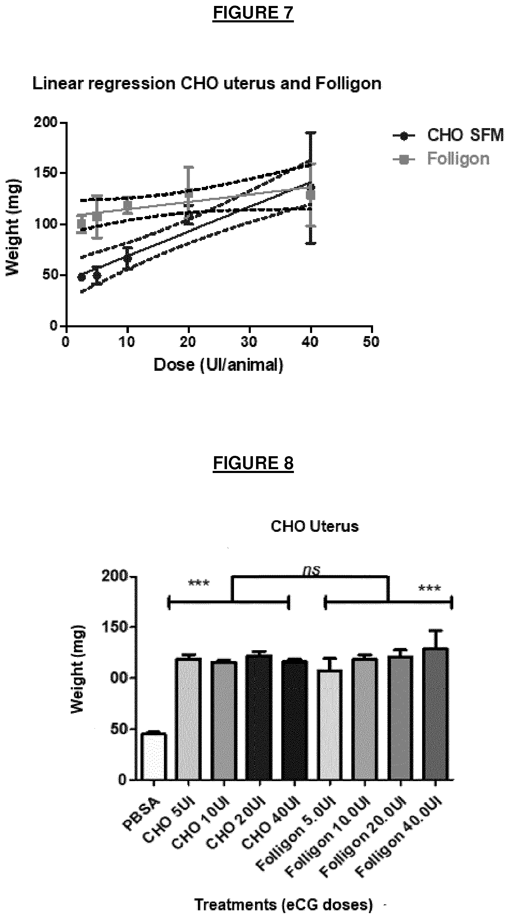

In Figure 7, there was a linear regression relating to uterine response to the administered hormone treatments. The abscissa axis shows the values related to injected dose (2.5 IU; 5 IU, 10 IU, 20 IU, and 40 IU) of reCG of the present invention or Folligon, or negative controls the PBSA and CHO-DG44 not producing βα and the ordinate axis shows the values related to weight (mg) of the reproductive organs dissected on the fourth day after treatment. In this analysis, the *slope* of the curve obtained for Folligon, 0.7263 ± 0.3767 (p = 0.0718) was not considered significant, while the *slope* obtained for the response to reCG of the present invention produced by CHO-DG44 cells was more consistent, 2.415 ± 0.4049 (p <0.0001). The controls showed no significant slopes. It was observed that the recombinant hormone produced by CHO-DG44 cells showed a dose-response trend better than the commercial product, Folligon. The last dose (40 IU) of reCG of the present invention showed a similar response to Folligon.

### Step 11: Purification of reCG of the present invention through chromatography

Two strategies were used for purification of reCG of the present invention contained in conditioned culture media of superproducing cells, namely: a) concanavalin A affinity chromatography (HiTrap™ ConA 4B, GE Healthcare); b) anion exchange chromatography (HiTrap™ Capto Q, GE Healthcare). The Äkta Purifier UPC-100 system (GE Healthcare) of high performance liquid chromatography (HPLC, High Performance Liquid Chromatography) associated with each one of the columns at a time, was used in optimization of reCG purification of the present invention, in independent procedures. The constant flow of 1 VC/min was used throughout the process, for both columns.

For the purifications performed with the HiTrap™ConA 4B column, the column was equilibrated with 3VC of buffer A (500 mM of NaCl, 20 mM of tris-HCl, 1 mM of MgCl2, 1 mM of CaCl2, pH 7,4). Then, 50 VC of conditioned medium containing 2X diluted reCGβα in dilution buffer (890 mM of NaCl, 40 mM of Tris-HCl, 2 mM of MgCl2, 0,2 mM of CaCl2, pH 7,4), filtered through 0,45 µm pore filters were applied to the column, followed by the column washing (5 VC) with buffer A. For elution, a linear methyl α-D-glucopyranoside concentration gradient (Sigma-Aldrich®) was applied, starting at 20 mM and ending at 300 mM (diluted in buffer A), at a range of 8VC. The permeate and wash fractions were collected, as well as the fractions of the eluates, and maintained at 4ºC.

For the purification carried out using the HiTrap™ Capto Q column, the column was equilibrated with 3VC of buffer A (10 mM NaCl, 100 mM Sodium Acetate, pH 4.5). Then, 20 VC of the conditioned medium containing 10X diluted reCGβα in dillution buffer A, filtered in 0,45 µm pore filters, and applied to the column, followed by washing the column (5 VC) with the buffer A. For the elution, different isocratic regimes of buffer B (1M of NaCl, 100 mM of Sodium acetate, pH 4.5) were applied: 0-11 mM of NaCl; 11-19 mM of NaCl; 11-19 mM of NaCl; 19-40 mM of NaCl, and completed in 1 M of NaCl, at a range of 12 VC. The permate and wash fractions were collected, as well as fractions of the eluates, have the pH adjusted for 7,4, and were kept at 4ºC.

For both types of performed chromatography, the fractions, permeate, washes, and eluates were diluted and subjected to ELISA for detection of reCGβα (*PMSG ELISA Kit*, ALPCO), following the manufacturer's protocol. The active fractions were concentrated using filters with 10kDa cut off (CentriconTM - Millipore®) and used for the following analysis: analysis of the purification process on polyacrylamide gels, using the *Coomassie Brilliant Blue* dye, and *in vivo* test (Figure 8).

Figure 8 illustrates *in vivo* biological activity of reCG of the invention partially purified by HiTrap™ Con A column, as from the uterine response of prepubertal female rats with25 days after treatment with Folligon or reCGβα produced in the absence of fetal calf serum bovine and partially purified by HiTrap™ Con A column. The abscissa axis shows the values related to performed treatments, consisting of doses (5, 10, 20 and 40 IU) of Folligon as the positive control or reCG of the invention produced by CHO-DG44 cells, and partially purified, and PBSA as the negative control; the ordinate axis shows the values related to weight (mg) of the reproductive organs dissected on the fourth day after treatment.

### Example 12: N-glycosylation study through NP-HPLC of N-glycans labeled with 2-AB and digestion with exoglycosidases

The fractions enriched in reCGβα obtained in Example 11 were concentrated about 100X using a Centricon device of 10 kDa cutoff (Millipore®) and applied on SDS-PAGE gels, as described in Example 11. Samples of 20 IUs of Folligon, and the 20 IUs of the international standard of eCG were also applied on SDS-PAGE gels, in order to perform the proposed comparison. The gels containing the samples to be analyzed were addressed to staining by Coomassie Blue (1,25 g of Coomassie R-250 Brilliant Blue diluted in 250 mL of methanol, 50 mL of glacial acetic acid and 200 mL of distilled water) for 16h. These gels were washed and bleached with solution containing 7.5% of acetic acid and 15% of methanol in distilled water. After bleaching, the bands present on eCG size range were excised from the gel individually cut with a scalpel, dried for 8 hours on a vacuum centrifuge at 30°C (SpeedVac) and processed through an appropriate protocol of ROYLE et al. (ROYLE et al., Determining the structure of oligosaccharides N- and O-linked to glycoproteins. Curr Protoc Protein Sci. 2006 Mar; Chapter 12:Unit 12.6).

The protocol consists of release the glycans with a gel digestion performed with PNGaseF and marked with 2-aminobenzene (*Glyko Signal 2-AB labeling kit,* Prozyme®). These samples of labeled glycans were then applied on an amide column (*Waters X-Bridge 3.5um amide column* of 4.6 x 250 mm), coupled to a pump (*Waters Binary Pump* 1525u) and subjected to normal phase chromatography using 2475 Waters system, being detected by a fluorescence detector (*Waters multi wavelength fluorescent detector,* excitation at 330nm, and emission at 420nm). All marked samples to be analyzed, as well as the standard of glucose units (*2-AB glucose ladder* - Prozyme ®) and the blank (Ultrafiltered Water, Milli-Q - Millipore ®) was diluted 5X in acetonitrile before application to the amide column. The used program is summarized in Table 2.

**Table 2: NP-HPLC Program used to analyze glycans of recombinant eCG βα of the invention and commercial eCG.**

| **Time (minutes)** | **Flow (mL/min)** | **Buffer A** | **Buffer B** |
|---|---|---|---|
| 0 | 0.86 | 20 | 80 |
| 48 | 0.86 | 50 | 50 |
| 49 | 0.86 | 100 | 0 |
| 53 | 0.86 | 100 | 0 |
| 55 | 0.86 | 20 | 80 |
| 63 | 0.86 | 20 | 80 |
| 64 | 0 | 20 | 80 |

| | | | |
|---|---|---|---|
| Buffer A: 50 mM of ammonium formate, pH 4.4. Buffer B: Acetonitrile. Column volume: 1 mL. | | | |

The calculation of the glucose units (GU) corresponding to each peak obtained was performed by comparing the retention time thereof versus the standard glucose curve (nine points) in a fifth-order polynomial distribution. Thus, it was possible to determine GUs for each obtained retention time corresponding to each peak of each sample.

Aliquots of glycan pool obtained for each sample were dried by vaccum centrifugation and resuspended in 2 µL of 5X glycosidase buffer (Prozyme®), added with 1 µL of an array of glycosidases and water q.s. up to 10µL-. The following combination of glycosidases in an incubation at 37°C for 16 h, was used to digest the glycan samples even their basal structures:
1. Sialidase (*Glyko Sialidase A TM- A. Urefaciens* - Prozyme);
2. Sialidase + Fucosidase (*Glyko β-Fucosidase™* - *Bovine Testis* - Prozyme);
3. Sialidase + Fucosidase + Galactosidase (Glyko β-Fucosidase™- Bovine Testis - Prozyme) and
4. Sialidase + Fucosidase + Galactosidase + Hexosaminidase (*Glyko Hexosaminidase™* - *Jack Bean* - Prozyme).

After digestion, these samples were purified in the glycan purification cartridge (*Glyko clean S* - Prozyme) and subjected again to HPLC under the same conditions described above.

Comparisons between putative structures of glycans present in eCG described in the literature, pre-characterized structures available in online database (*Glycobase* 3.2) and GUs obtained by HPLC allow a quantitative and qualitative analysis of the glycans released by performed successive digestions.

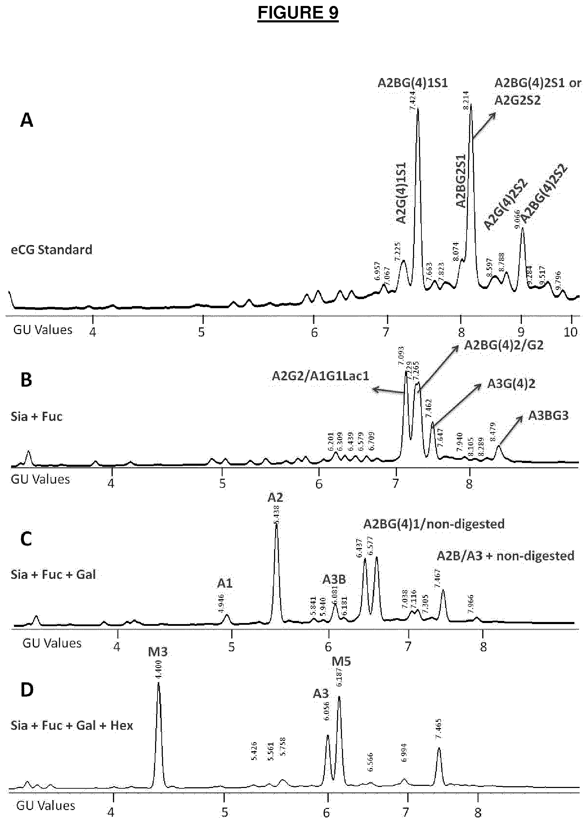

Figure 9 shows the putative structures selected for each peak, after comparison with the pattern of digestion performed with exoglycosidades. In the abscissas axis there are the relative values of glucose units (GU) obtained for each chromatography, while on the ordinate axis there are the intensities of the eluted peaks (EU). In panel A, there are the undigested structures, and in the B, C and D panels there are the structures obtained after digestion with sialidase, sialidase + galactosidase + fucosidase, and sialidase + galactosidase + fucosidase + hexosaminidase, respectively.

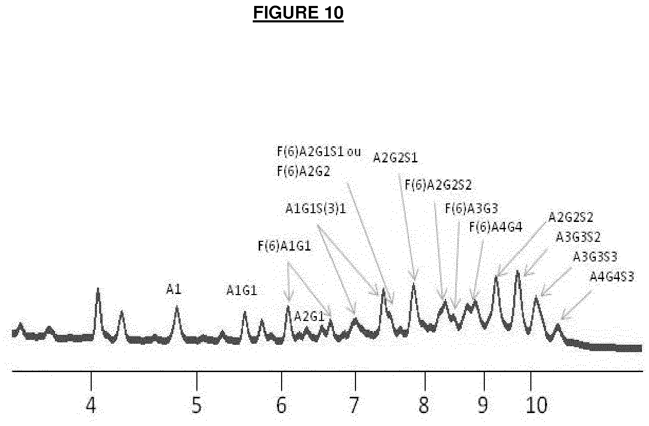

Figure 10 shows the putative N-glycan structures obtained in fractions enriched in reCG of the invention. In the abscissa axis, values are found for glucose units (GU) obtained for each chromatography, while on the ordinate axis there are the intensities of the eluted peaks (EU). The arrows indicate potential N-glycan structures assigned to each peak obtained for reCGβα expressed in CHO-DG44 strain. After analysis of N-glycosylation profile of reCG, it was possible to verify a majority presence of peaks corresponding to di- or tri-galactosylated N-glycans, these subsequently being mono-, di- or tri-sialylated. This is in accordance with the N-glycan profile observed for the commercial eCG Folligon (Figure 9).

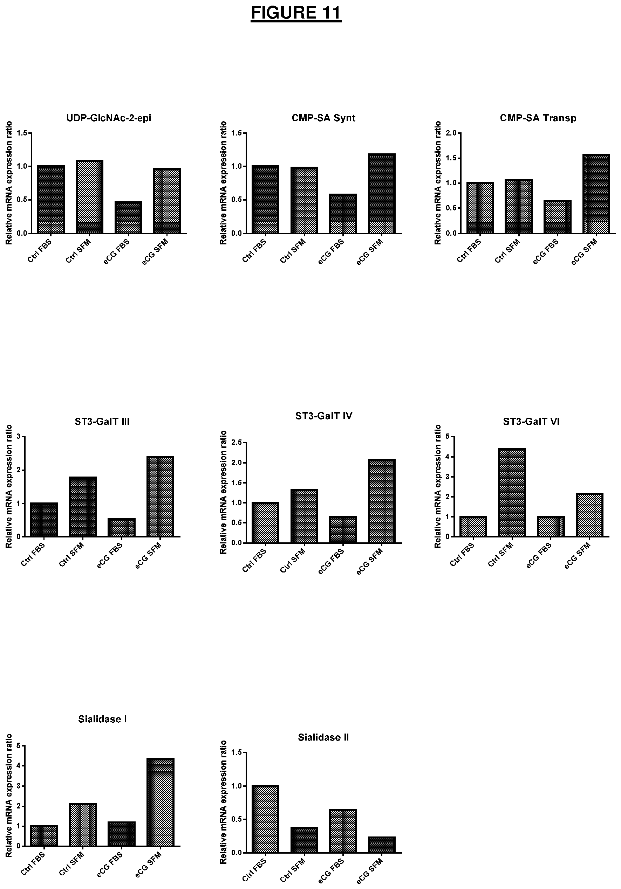

Figure 11 shows gene expression results for the transcripts corresponding to enzymes of the glycosylation pathways in control cells (non-producing CHO cells of eCG called Ctrl), and producing cells of eCG (called eCG), both cultured in the presence of fetal calf serum (FCS) or in its absence (SFM - *serum-free medium*). Gene expression was analyzed by quantitative real-time (qRT-PCR) PCR (*Polymerase Chain Reaction*) using *GAPDH* (Glyceraldehyde-3-Phosphate Dehydrogenase) gene as normalizer. The analyzed targets were: UDP-GlnNAc-2-epimerase, CMP-AS-synthetase, CMP-SA-transporter, β-galactosidase α 2,3-sialyltransferase 3 (ST3-GalTIII), β-galactosidase α 2,3 sialyltransferase 4 (ST3-GalTIV), β-galactosidase α 2,3 sialyltransferase 6 (ST3-GalTVI), Sialidase I, Sialidase II. It was possible to obtain the increased expression of the transcripts of UDP-GlnNAc-2-epimerase, CMP-AS-synthetase, CMP-SA-transporter, β-galactosidase α 2,3-sialyltransferase 3 (ST3- GalTIII), β-galactosidase α 2,3-sialyltransferase 4 (ST3-GalTIV), β-galactosidase α 2,3-sialyltransferase 6 (ST3-GalTVI) and Sialidase I.

### Example 12: Clinical trial performed in cows in anoestrus

A clinical trial was performed in cows in anoestrus with reCGβ product. The study included 45 *Bos indicus* female cattle clinically healthy, weighing between 350 and 600 kg, of childbearing age between 3 and 10 years. The animals were kept in *Brachiaria brizantha* picket with a capacity of 1 AU/ha in continuous grazing period during the experimental period and had free access to water and mineral salt.

The study included animals that did not show reproductive disorders; not received any reproductive procedure (insemination, cover or collection of embryo) 20 days before the beginning of the experiment; they were clinically healthy at the beginning of the experimental period, and received no treatment of disease within 10 days prior to the study; did not receive administration of any hormonal product during the 30 days prior to the study; had a body condition scoring above 2.0 (scale of 1 to 5, AYRES et al., Validation of body condition score as a predictor of subcutaneous fat in Nelore (Bos indicus) cows Livest. Sci. 2009; 123:175-179). To be included in the study one of the animal ovaries should present dominant follicle.

The recruited 45 females, divided into three treatment groups (reCGβα-CHO-DG44, Positive Control and Negative Control as Folligon), so that each group contained 15 animals. The distribution of treatment was performed in randomized way by composite block design (CBD) within each category of follicular diameter (<8 mm, between 8 to 10 mm, 10 to 12 mm and > 12 mm). The groups of treated animals with the CB050formulations prepared through concentration of the protein contained in the supernatant of the cell culture by ultrafiltration, formulation of the concentrate with lactose, mannitol, and methylparaben; e lyophilization, and positive control received a single dose of 300 IU/animal, intramuscularly, 8 days after the beginning of ovulation synchronization protocol. While the negative control group of animals received a single dose of 1.5mL of sterile saline.

On the baseline date of study (D0), the animals were evaluated for body condition scoring, general health status, and reproductive assessment via ultrasound. On the same day (D0), the females were previously synchronized. The synchronization consisted of intramuscular administration of 2 mg of estradiol benzoate and inserting an intravaginal progesterone device. Eight days later (D8), the progesterone intravaginal device was removed, and all animals received intramuscularly 500µg cloprostenol (PGF) and 1 mg estradiol cypionate. At this time, the animals were distributed into groups where they received treatments (300 IU of reCG of the invention; Folligon of 300 IU or 1,5 mL of negative control).

The ultrasound evaluations (Kai Xin, KX 5100, Xuzhou KaiXin Electronic Instrument Company Ltd., China; Figure 6) were performed in all study animals in timepoints: animal selection (D0), on D8 to evaluate the follicle dominant, and separation of the treatment groups; on D10 to evaluate the follicular growth between D8 and D10; and on D18 to evaluate ovulation rate and diameter of the *corpus luteum.*

**Table 3: Clinical Study of reCG of the invention in Nelore cows in anoestrus.**

| **CLINICAL TRIAL** | **Control** | **reCGCHO** | **Folligon** | **P** |
|---|---|---|---|---|
| Number of animals | 15 | 15 | 15 | |
| Body Condition Scoring | 2.6±0.1 | 2.7±0.1 | 2.6±0.1 | 0.94 |
| Weight (Kg) | 443.7±3.4 | 439.3±7.8 | 434.0±6.0 | 0.67 |
| Age (months) | 75.2±2.2 | 76.8±3.1 | 78.4±2.6 | 0.88 |
| Ø follicular on D8 (mm) | 10.4±0.9 | 10.4±0.9 | 9.6±0.6 | 0.91 |
| Ø follicular on D10 (mm) | 10.7±1.0 | 12.6±0.7 | 12.5±0.8 | 0.27 |
| Follicular Growth from D8 to D10 (mm) | **0.4±0.1 ^{b}** | **1.4±0.3^{a}** | **1.5±0.3^{a}** | **0.01** |
| Ø of CL on D18 (mm) | 20.6±0.8 | 21.1±0.6 | 20.9±0.6 | 0.86 |
| Ovulation Rate (%) | 60.0 (9/15) | 86.7 (13/15) | 86.7 (13/15) | 0.07 |

It should be noted that the dominant follicle diameter of different experimental groups had, on average, similar size (P = 0.91), enhancing the homogeneity of distribution of different sized follicles between treatments. It was found that the cows of Folligon and reCGβα groups had end growth of the dominant follicle (P = 0.01) greater than the control group. Similar results were observed by SALES et al. (SALES et al., Fixed-time Al protocols replacing eCG with a single dose of FSH were less effective in stimulating follicular growth, ovulation, and fertility in suckled-anestrus Nelore beef cows. Anim Reprod Sci. 2011 Mar;124(1-2):12-8), wherein the eCG treatment promoted greater follicular growth (eCG - 1.40 mm/day vs. No eCG - 0.95 mm/day). There was no difference between experimental groups on the diameter of the dominant follicle at D10 (P = 0.27), on ovulation rate (P = 0.07) and on the diameter of the *luteum corpus* (P = 0.86).

## Claims

1. PROCESS FOR PRODUCING A RECOMBINANT EQUINE CHORIONIC GONADOTROPIN, **characterized by** the steps of:
(a) inserting a DNA sequence encoding an eCG in an expression vector including encoding cDNA of the enzyme dihydrofolate reductase (DHFR);
(b) co-transfecting the vector of step (a) with a selection vector in double negative expression cells for the DHFR gene;
(c) selecting transfected cells resistant to antibiotic treatment to which they shall presents resistance;
(d) gene amplification of the expression vector and selection of superproducing cells of reCG by treatment with increasing concentrations of the DHFR inhibitor;
(e) growing the superproducing cells of reCGαβ with gene expression profile determined for transcripts of enzymes of the glycosylation pathways for expression of reCGαβ;
(f) purifying of the obtained reCGαβ.

2. PROCESS, according to claim 1, **characterized in that** the DNA sequence encoding an eCG comprises a chain of fused cDNA containing sequences encoding both α and β chains of equine chorionic gonadotrophin (eCG).

3. PROCESS, according to claim 1, **characterized in that** the DNA sequence encoding an eCG comprises cDNA chains containing sequences encoding α and β chains separately.

4. PROCESS, according to claim 1, **characterized in that** the DNA sequence encoding an eCG is SEQ ID NO: 1.

5. PROCESS, according to claim 1, **characterized in that** the expression vector is the pNUØ vector.

6. PROCESS, according to claim 1, **characterized in that** the expression cells are chosen from mammalian cell line, insect cells or any cell type comprising certain gene expression profile for the transcripts of enzymes of the glycosylation pathways.

7. PROCESS, according to claim 6, **characterized in that** the expression cells are CHO line cells, BHK, 293, Vero or derivatives thereof.

8. PROCESS, according to claim 7, **characterized in that** the cells are expression cells are CHO-DG44 line cells.

9. PROCESS, according to claim 1, **characterized in that** the selection vector is pX343 vector.

10. PROCESS, according to claim 1, **characterized in that** the ratio of the expression vector to selection vector in co-transfection step ranges from 1:1 to 1:5000.

11. PROCESS, according to claim 10, **characterized in that** said ratio ranges between 1:40 and 1:120.

12. PROCESS, according to claim 11, **characterized in that** said ratio is 1:40.

13. PROCESS, according to claim 1, **characterized in that** the DHFR inhibitor is methotrexate.

14. PROCESS, according to claim 1, **characterized in that** said enzymes of the glycosylation pathway are UDP-GlnNAc-2-epimerase, CMP-SA-sintetase, CMP-SA-transporter, β-galactosidase α 2,3-sialyltransferase 3 (ST3-GalTIII), β-galactosidase α 2,3-sialyltransferase 4 (ST3-GalTIV), β-galactosidase α 2,3-sialiltransferase 6 (ST3-GalTVI), Sialidase I, Sialidase II.

15. PROCESS, according to claim 1, **characterized in that** said culture of superproducing cells of reCG occurs in culture medium containing from about 2 to 10% of fetal calf serum or chemically defined medium supplemented with growth factors and precursors used in the glycosylation pathways.

16. PROCESS, according to any one of claims 1 to 15, **characterized in that** the cells with said gene profile for the enzyme transcript of the glycosylation pathway are those which ensure the biological activity of the reCG in target species.

17. PROCESS, according to any one of claims 1 to 15, **characterized in that** it enables an expression rate of reCG ranging from 1 to 100 mg/L.

18. PROCESS, according to claim 1, **characterized in that** the purification step is performed by at least one of ion exchange chromatography and affinity chromatography, protein precipitation, ultrafiltration or tangential filtration.

19. RECOMBINANT EQUINE CHORIONIC GONADOTROPIN, **characterized in that** it is produced by the process defined in claims 1 to 18.

20. VETERINARY COMPOSITION comprising:
a) a veterinary effective amount of reCG defined by claim 19;
b) optionally additives; and
c) a pharmaceutically acceptable carrier.

21. USE OF RECOMBINANT EQUINE CHORIONIC GONADOTROPIN, defined in claim 19, or veterinary composition containing said gonadotropin, **characterized in that** it is for the manufacture of a medicine for the veterinary conditions treatment and reproduction-related protocols and mammalian ovulation.

22. USE OF RECOMBINANT EQUINE CHORIONIC GONADOTROPIN, according to claim 19, or composition containing said gonadotropin **characterized in that** it is for the manufacture of diagnostic kits for veterinary conditions, and reproduction-related protocols and mammalian ovulation.

23. USE OF RECOMBINANT EQUINE CHORIONIC GONADOTROPIN, according to claim 19, or composition containing said gonadotropin, **characterized in that** it is for the manufacture of supplement for cell culture.
